# EUROPEAN PATENT SPECIFICATION

(11) **EP 1 806 136 B1**
(45) Date of publication and mention of the grant of the patent: **30.11.2011**
(21) Application number: 05790245.4
(22) Date of filing: 04.10.2005
(51) Int. Cl.: A61K 31/4045, A61P 13/08, C07D 209/14

(54) **MEDICINAL COMPOSITION FOR PREVENTION OF TRANSITION TO OPERATIVE TREATMENT FOR PROSTATIC HYPERTROPHY**
MEDIZINISCHE ZUSAMMENSETZUNG ZUR PRÄVENTION DES ÜBERGANGS ZU EINER OPERATIVEN BEHANDLUNG BEI PROSTATAHYPERTROPHIE
PRÉPARATION THÉRAPEUTIQUE VISANT À ÉLIMINER L'ÉTAPE DE TRANSITION VERS LE TRAITEMENT OPÉRATOIRE DE L'HYPERTROPHIE PROSTATIQUE

(30) Priority: 06.10.2004 JP 2004294358; 01.06.2005 JP 2005160843
(43) Date of publication of application: 11.07.2007
(73) Proprietor: Kissei Pharmaceutical Co., Ltd., Matsumoto-shi Nagano 399-8710 (JP)
(72) Inventor: OKUBO, Yoshio, Kissei Pharmaceutical Co., Ltd., Tokyo 112-0002 (JP); SHIMIZU, Tomoji, Kissei Pharmaceutical Co., Ltd., Tokyo 112-0002 (JP); ARAI, Nobuhiko, Kissei Pharmceutical Co., Ltd., Tokyo 112-0002 (JP); OMORI, Yasuhiro, Kissei Pharmaceutical Co., Ltd., Tokyo 112-0002 (JP)
(74) Representative: Hayes, Adrian Chetwynd
(86) International application number: PCT/JP2005/018356
(87) International publication number: WO 2006/038619

(56) References cited:
- EP-A- 1 541 554
- WO-A-2005/027923
- WO-A-2005/085195
- DATABASE EMBASE [Online] ELSEVIER SCIENCE PUBLISHERS, AMSTERDAM, NL; 2001, SORBERA L A ET AL: "KMD-3213. Treatment of BPH, [alpha]1-adrenoceptor antagonist" XP002462575 Database accession no. EMB-2001338890 & DRUGS OF THE FUTURE 2001 SPAIN, vol. 26, no. 6, 2001, pages 553-560, ISSN: 0377-8282
- ISHIGURO MASAJI ET AL: "Identification of binding sites of prazosin, tamsulosin and KMD-3213 with alpha(1)-adrenergic receptor subtypes by molecular modeling." 11 October 2002 (2002-10-11), LIFE SCIENCES 11 OCT 2002, VOL. 71, NR. 21, PAGE(S) 2531 - 2541 , XP002463593 ISSN: 0024-3205 p. 2533 table 2 and p. 2540 last parag. of discussion
- DATABASE INTEGRY Prous; J Urol 2005, 173(4, Suppl.): Abst 1642 26 May 2005 (2005-05-26), YOSHIDA M. ET AL.: "Silodosin, a new effective alpha1A-adrenoceptor selective antagonist for the treatment of benign prostatic hyperplasia: Results of a phase 3 randomized, placebo-controlled, double-blind study" XP002462492
- O'LEARY M P: "Lower urinary tract symptoms/benign prostatic hyperplasia: Maintaining symptom control and reducing complications" UROLOGY 2003 UNITED STATES, vol. 62, no. 3 SUPPL. 1, 2003, pages 15-23, XP002462573 ISSN: 0090-4295 1527-9995
- KIRBY R S ET AL: "Efficacy and tolerability of doxazosin and finasteride, alone or in combination, in treatment of symptomatic benign prostatic hyperplasia: The Prospective European Doxazosin and Combination Therapy (PREDICT) trial" UROLOGY 01 JAN 2003 UNITED STATES, vol. 61, no. 1, 1 January 2003 (2003-01-01), pages 119-126, XP002462574 ISSN: 0090-4295
- YOKOYAMA OSAMU ET AL: 'Shin'yaku Tenbo 2004 Dai III Bu Chiryo ni Okeru Saikin no Shin'yaku no Ichizuke- Shin'yaku no Hiroba- Hainyo Chikunyo Shogai Chiryoyaku.' MEDICINE AND DRUG JOURNAL. vol. 40, 31 January 2004, pages 590 - 594, XP002999869
- Supervised by Yasuahi TSUKAMOTO, "Zenritsusen Haidaisho" CURRENT PHARMACY, 2002.03, No 13, pages 1 to 5, 13. XP002999870

## Description

### Technical Field

The present invention relates to a pharmaceutical composition for the prevention of transition to surgical therapy for benign prostatic hyperplasia.

More particularly, the present invention relates to a pharmaceutical composition for the prevention of transition to surgical therapy for benign prostatic hyperplasia, which comprises an indoline derivative, namely silodosin or a pharmaceutically acceptable salt thereof.

### Background Art

Benign prostatic Hyperplasia is a disease which has high generation frequency in aged males, and it is said that 70% of males of sixties are suffering from this in the aging society of recent years, the number of patients of benign prostatic hyperplasia has been increasing and drawing great attention.

Main therapeutic methods for benign prostatic hyperplasia are drug therapy and surgical therapy. As the surgical therapies, open prostatectomy, transurethral resection of the prostate (TUR-P), thermotherapy, laser therapy, stent indwelling method and the like are known. The open prostatectomy becomes indication in the case of large size of prostate, in the case of repeated urinary retention and the like, but is most invasive. TUR-P is a method in which an endoscope is inserted from urethra, and the prostate is cut off with an electric surgical knife from the inside while observing the prostate using a mirror. Currently, this is the most popularized standard method as the surgical therapy for benign prostatic hyperplasia, which is carried out on about fifty thousand cases a year in Japan and about three hundred thousand cases a year in the United States. In comparison with the open prostatectomy, bleeding is little, and recovery after the operation is quick. Also, the thermotherapy is a method in which opening of the urethra is improved by destroying tissue of the prostatic inner gland by heating them through the irradiation of microwave from the urethra. This is a therapeutic method which can be carried out for out-patients, causes little pain and is effective on the benign prostatic hyperplasia of moderate or less severity. The laser therapy is a method in which the tissue of prostatic hypertrophy adenoma is allowed to undergo necrosis or is destroyed by irradiating laser. Since the method has many advantages in comparison with TUR-P, such as little bleeding, short treating period of time, little pain in patients and the like, this is drawing attention as a highly advanced therapeutic method ranking with the thermotherapy. The stent indwelling method is a method in which at duct (stent) is indwelled in a urethral part thinned by the pressure of swollen prostate (e.g., see Non-patient Reference 1).

As the drug therapy for benign prostatic hyperplasia, an αl adrenoceptor antagonist, an anti-androgen drug, a herb medicines and the like are used. It has been reported that the effect of an al adrenoceptor antagonists tamsulosin hydrochloride, to reduce storage disorder is indicated to be similar to that of TUR-P an short terms (e.g., see Non-patent Reference 2).

In deciding the therapeutic method, in general, overall severity based on I-PSS. (International Prostate Symptom Score) total score, QOL score, maximum flow rate, residual urine volume and prostate volume is used. For example, when the overall severity is moderate or more, surgical-therapy such as the TUR-P or the like become the indication (e.g., Non-patent References 3 and 4). However, since there are many patients who do not desire the surgical therapy as an invasive treatment, the development of a drug which can prevent the transition to a surgical therapy in severe patients who are subject to the surgical therapy has been desired.

Silodosin or a pharmaceutically acceptable salt thereof is markedly useful as an agent for the treatment, of dysuria associated with benign prostatic hyperplasia and the like, because it has a selective inhibitory activity against urethral smooth muscle contraction and can lower urethral pressure without great influence upon blood pressure (see Patent References 1 to 3). However, there are no descriptions or suggestions therein that silodosin of the following formula (II), or pharmaceuticallly acceptable salts thereof, can prevent the transition of a patient with benign Prostatic hyperplasia who is subject to surgical therapy to the surgical therapy. 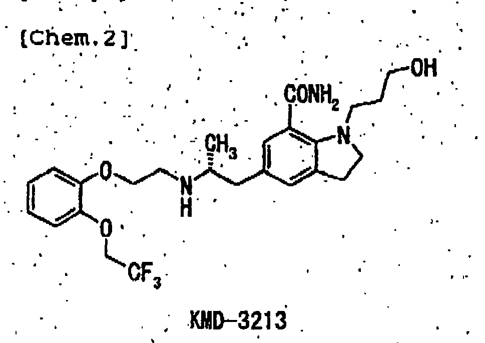
Patent Reference 1 JP-A-H06-220015;
Patent Reference 2: International Publication No. 99-15202 pamphlet;
Patent Reference 3: International Publication No. 2004-22538 pamphlet;
Non-patent Reference 1: Satoru Takahashi, "Yoku Wakaru Zenritsusen No Byoki (Good Understanding on Prostatic Diseases)", published by Iwanami Shoten, March 4, 2004, pp. 58-94;
Non-patent Reference 2: B. Djavan, Shinyaku To Chiryo (New Drugs and Treatments), No. 442, vol. 53, no. 3, 2003, pp. 11-12;
Non-patent Reference 3: Hainyo Shogai Rinsho Shiken Gaidolain Sakusei Iinkai Hen (Edited by Urinary Disturbance Clinical Test Guideline Preparation Committee), "Hainyo, Shogai Rinsho Shiken Gaidolain Daiichibu Zenritsusen HidaiSho (Urinary Disturbance Clinical Test Guideline Part 1, Benign Prostatic Hyperplasia", published by Igaku Tosho Shuppan, 1997;
Non-patent Reference 4: Hinyokika Ryoiki No Chiryo Hyojunka Ni Kansuru Kenkyu Han Hen (Edited by a Research Group on the Treatment Standardization in the Field of Urology), "Zenritsusen Hidaisho Shinryo Gaidolain (Guideline on the Medical Examination and Treatment of Benign Prostatic Hyperplasia)", published by Jiho Shuppan, 2001, pp. 11-24.

### Disclosure of the Invention

### Problem to be solved by the Invention

The object of the present invention is to provide an agent which can prevent the transition of benign prostatic hyperplasia to a surgical therapy.

### Means of Solving the Problems

Taking the above problems into consideration, the present inventors have conducted intensive studies and surprisingly found that by administering silodosin or a pharmaceutically acceptable salt thereof, transition to the surgical therapy can be prevented in patients with benign prostatic hyperplasia who are subject to a surgical therapy, thereby forming the basis of the present invention.

Thus, the present invention provides a pharmaceutical composition for oral administration, for use in the prevention of transition to surgical therapy for benign prostatic hyperplasia in a patient of benign prostatic hyperplasia whose overall severity is severe, which comprises as daily dose per adult 4 to 8 mg of silodosin or a pharmaceutically acceptable salt thereof.

The present invention also provides a pharmaceutical composition for oral administration for use in treating a patient of benign prostatic hyperplasia whose overall severity is severe, which comprises as daily dose per adult 4 to 8 mg of silodosin or a pharmaceutically acceptable salt thereof.

The present invention also provides use of silodosin or a pharmaceutically acceptable salt thereof in the manufacture of a pharmaceutical composition for oral administration comprising as daily dose 4 to 8 mg per adult of the silodosin or the pharmaceutically acceptable salt thereof for preventing the transition to surgical therapy for benign prostatic hyperplasia in a patient of benign prostatic hyperplasia whose overall severity is severe or for treating a patient of benign prostatic hyperplasia whose overall severity is severe.

The surgical therapy may be transurethral resection of the prostate.

The pharmaceutical composition for the prevention of transition to surgical therapy for benign prostatic hyperplasia may be administered to a patient of benign prostatic hyperplasia whose overall severity is severe.

The daily dose of the silodosin or pharmaceutically acceptable salt thereof is from 4 to 8 mg.

### Effect of the Invention

The pharmaceutical composition which comprises silodosin or a pharmaceutically acceptable salt thereof is useful as an agent for the prevention of transition to surgical therapy for a patient of benign prostatic hyperplasia who is subject to surgical therapy.

### Best Mode to Practice the Invention

The silodosin, namely (-)-1-(3-hydroxypropyl)-5-((2R)-2-{[2-({2-[(2,2,2-trifluoroethyl)oxy]phenyl}oxy)ethyl]amino}propyl)-2,3-dihydro-1H-indole-7-carboxamide, can be prepared by the method described in Patent Reference 2.

Solvates of the silodosin with pharmaceutically acceptable solvents such as water or ethanol are also included.

As the pharmaceutically acceptable salts of the silodosin, for example, a carboxy group may be converted into its salt with an inorganic base such as sodium, potassium or calcium or with an organic amine such as morpholine or piperidine.

The pharmaceutical composition for the prevention of transition to surgical therapy for benign prostatic hyperplasia can be prepared by mixing the silodosin or pharmaceutically acceptable salt thereof with commonly used drug preparation carriers.

The drug preparation carriers may be used by optionally combining them depending on administration forms, and their examples include excipients such as lactose; lubricants such as magnesium stearate; disintegrating agents such as carboxymethylcellulose; binders such as hydroxypropylmethylcellulose; surfactants such as macrogol; foaming agents such as sodium bicarbonate; dissolving aids such as cyclodextrin; acidity agents such as citric acid; stabilising agents such as sodium edetate; and pH adjusting agents such as phosphate.

As the administration form of the pharmaceutical composition for the prevention of transition to surgical therapy for benign prostatic hyperplasia, for example, oral administration preparations such as powders, granules, fine subtilaes, dry syrups, tablets, or capsules can be illustrated.

The above preparations are prepared in such a manner that the silodosin or a pharmaceutically acceptable salt thereof is administered within the range of from 4 to 8 mg, per day per adult, as the oral administration preparations.

In the present invention, the patients of benign prostatic hyperplasia who become the object of indication are patients with a severity which becomes the indication object of surgical therapy, namely severe, excluding those patients who have urinary retention or complications caused by benign prostatic hyperplasia, and for example, patients of severe based on the criteria for judging overall severity of the above guideline on the medical examination and treatment or benign prostatic hyperplasia can be illustrated.

In the present invention, the surgical therapy for benign prostatic hyperplasia means an invasive treatment which is carried out with the aim of treating benign prostatic hyperplasia, and a low invasive treatment is also included therein. For example, open prostatectomy, transurethral resection of the prostate (TUR-P), thermotherapy, laser therapy, stent indwelling method and the like can be illustrated, and TUR-P is particularly preferable.

In the present invention, the prevention of transition to surgical therapy means a therapy.which is carried out with the aim of avoiding the surgical therapy, or prolonging operating time of the surgical therapy, in the above patients who are subject to the surgical therapy.

### Examples

The present invention is further illustrated in more detail by way of the following Examples. However, the present invention is not limited hereto.

### [Example 1]

### Clinical effects in patients who are subject to surgical therapy for benign prostatic hyperplasia.

Based on the results of a placebo-controlled, parallel, groups-comparing double blind trial carried out in patients of benign prostatic hyperplasia, clinical effects of silodosin and tamsulosin hydrochloride in patients who are subject to surgical therapy were evaluated. As the patients who are subject to surgical therapy, patients whose overall severity before administration was "severe" based on the criteria for judging overall severity (Non-patient Reference 3) were extracted.
Objects: 147 patients who are subject to surgical therapy for benign prostatic hyperplasia
Administration method: oral administration for 12 weeks.
Administered groups: Silodosin group (4 mg silodosin/once, twice a day, 63 patients), Tamsulosin group (0.2 mg tamsulosin hydrochloride, once a day, 55 patients) and Placebo group (placebo drug, 29 patients).
Primary endpoints: I-PSS total score, QOL score
Criteria for judging overall severity: in the severity of respective judging items shown in Table 1, the overall severity is regarded as "severe"' when "severe" is present in 2 items or more. In this connection, the Qmax in the table means maximum flow rate (mL/second), RU means residual urine volume (mL) and PV means estimated prostate volume (mL) by ultrasonic tomography.

### [Table 1]

**Table 1. Criteria for judging overall severity**

| Respective judging items | Moderate | Severe |
|---|---|---|
| 1. T-PSS total score | 8 - 19 | 20-35 |
| 2. QOL score | 2, 3, 4 | 5, 6 |
| 3. Qmax; Residual urine volume | Qmax: 5 mL/sec or more and RU: less than 100 mL | Qmax: less than 5 mL/sec or RU: 100 mL or more |
| 4. Estimated prostate volume (PV) | 20 mL or more, and less than 50 mL | 50 mL or more |

### I-PSS items questioned

### (1) Sensation of residual urine

"Have you had a sensation of not emptying your bladder completely after you finished urinating?"

### (2) Urination within 2 hours

Have you had to urinate again less than two hours after you finishes urinating?"

### (3) Intermittency of urinary stream

"Have you found you stopped and started again-several times when you urinated?"

### (4) Urgency

"Have you found it difficult to postpone urination?"

### (5) Power of urinary stream

"Have you had a weak urinary steam?"

### (6) Straining during urination

"Have you had to push or strain to begin urination?"

The scorers of (1) to (6) are as follows.

Not at all: 0 point, less than 1 time in 5 : 1 point, less than 1 time in 2: 2 points, about 1 time in 2: 3 points, more than 1 time in 2: 4 points, almost always: 5 points.

### (7) Nocturia

"How many times did you get up to urinate from the time you went to bet at night until the time you got up in the morning?"

The scores are as follows.

None: 0. point, 1 time: 1 point, 2 times: 2 points, 3 times: 3 points, 4 times: 4 points, 5 times or more: 5 point.

### QOL score

"How do you feel if the present condition of urination will continue from now on?"

Very satisfied: 0 point, satisfied : 1 point, generally satisfied: 2 points, neither satisfied nor unsatisfied : 3 points, a litle unsatisfied : 4 points, unsatisfied: 5 points, very unsatisfied: 6 points.

### Results

regarding the case of patients whose overall severity before administration was severe based on the criteria for judging overall severity (Non-patent Reference 3), changes in I-PSS total score and QOL score at the final evaluation were compared. The results are shown in Table 2 and Table 3, respectively. In this connection, comparison between the administration groups was carried out by two sample t-test, and in the tables, "*" and "**" show that significant differences were found at significance levels of less than 5% and less than 1%, respectively, and "N.S." means that a significance was not found.

### [Table 2]

**Table 2. Change in I-PSS total score**

| Administration groups | Cases | I-PSS before administration | Change in I-PSS at the end |
|---|---|---|---|
| | | | Mean ± standard deviation |
| Silodosin | 63 | 22.8 | -12.0 ± 7.1 ^{1) * 2) **} |
| Tamsulosin | 55 | 21.9 | -8.6 ± 6.2 ^{3) N.S.} |
| Placebo | 29 | 23.5 | -8.2 ± 8.2 |

| | | | |
|---|---|---|---|
| 1) Silodosin group vs. Placebo group 2) Silodosin group vs. Tamsulosin group 3) Tamsulosin group vs. Placebo group *: P < 0.05 ; **: P < 0.01; N.S.: no significance | | | |

### [Table 3]

**Table 3. Change in QoL score**

| Administration groups | Cases | QOL before administration | Change in QOL at the end |
|---|---|---|---|
| | | | Mean ± SD |
| Silodosin | 63 | 5.5 | -2.3 ± 1.6. ^{1) ** 2) **} |
| Tamsulosin | 55 | 5.3 | -1.5 ±1.3 ^{3) N.S.} |
| Placebo | 29 | 5.3 | -1.4 ± 1.3 |

| | | | |
|---|---|---|---|
| 1) Silodosin: group vs. Placebo group 2) silodosin group vs. Tamsulosin group 3) Tamsulosin group vs. Placebo group **: P < 0: 01; N.S.: no significance | | | |

As shown in Table 2, the Silodosin group significantly improved the I-PSS total score in the patients of benign prostatic hyperplasia whose overall severity before administration was severe. The effect of the Silodosin group was statistically significant in comparison with the placebo group and Tamsulosin group. In addition, as shown in Table 3, the Silodosin group also improved QOL score statistically significantly in comparison with the Placebo group and Tamsulosin group. Based on this, it was shown that silodosin significantly improves I-PSS total score in patients of benign prostatic hyperplasia who are subject to surgical therapy and has an effect to avoid transition to surgical therapy.

Next, the effect of each administration group to improve the severity of I-PSS total score was evaluated in the patients of benign prostatic hyperplasia whose overall severity before administration was severe. The results are shown in Table 4. In this connection, comparison between the administration groups was carried out by two sample wilcoxon test, and in the able, "*" and "**" show that significant differences were found at significance level of less than 5% and less than 1%; respectively, and "N.S." means that a significance was not found.

### [Table 4]

**Table 4. Improving effect on severity of I-PSS total score**

| Administra ion groups | Cases | Severity of subjective symptoms at the Number of patients (%) | | | Comparison between groups |
|---|---|---|---|---|---|
| | | Mild (0 - 7) | Moderate (8 - 19) | Severe (20 35) | |
| Silodosin | 63 | 24 (38.1%) | 33 (52.4%) | 6 (9.5%) | 1) ** 2) * |
| Tamsulosin | 55 | 12 (21.8%) | 34 (61.8%) | (16.4%) | 3) N.S. |
| Placebo | 29 | 6 (20.7%) | 11 (37.9%) | 12 (41.4%) | |

| | | | | | |
|---|---|---|---|---|---|
| 1) Silodosin group vs. Placebo group 2) Silodosin group vs. Tamsulosin group 3) Tamsulosin group vs. Placebo group *: P < 0.05; **: P < 0.01; N.S.: no significance | | | | | |

As shown in Table 4, the silodosin group significantly improved the severity of I-PSS total score in the patients of benign prostatic hyperplasia whose overall severity before administration was severe. That is, it can be seen that the severity of I-PSS total score in about 38% of the patients who were subject to surgical therapy was improved to mild. The improving effect of the Silodosin group was a statistically significant difference in comparison with the Placebo group and Tamsulosin group.

As shown above, in the placebo-controlled, double blind, comparative clinical test, silodosin was able to significantly improve I-PSS total score and QOL score of the patients of benign prostatic hyperplasia whose overall severity before administration was severe and to improve symptoms of the patients who are subject to surgical therapy to a level outside of surgical-therapy object, and thereby to avoid transition to surgical therapy.

### [Example 2]

### Effects on the objective symptoms of patients who are subject to surgical therapy for benign Prostatic hyperplasia.

Based on the results from a long-term administration test in patients of benign-prostatic hyperplasia, the effects of silodosin on objective symptoms of patients who are subject to surgical therapy were examined. As the patients who are subject to surgical therapy, patients whose overall severities before administration were "moderate" and "severe" according to the criteria for judging overall severity described in the above Table 1 were extracted.
Objects: 229 patients who are Subject to surgical therapy for benign prostatic hyperplasia
Administration method: 4 mg silodosin/once (can be optionally reduced to 2 mg/once); twice a day, oral administration for 52 weeks
Analyzed groups: 101 severe patients (reduced to 2 mg/once in 9 cases among them), 128 moderate patients (reduced to 2 mg/once in 17 cases among them)

As an objective symptom, results of maximum flow rate (Qmax) (mL/sec) are shown in Table 5.

### [Table 5]

**Table 5. Improving effect on maximum flow rate (Qmax)**

| Groups | Administra tion group | Cases | Before administration (mean ± SD) | Change after 52 weeks (mean ± SD) |
|---|---|---|---|---|
| Severe | silodosin | 101 | 8.9 ± 3.0 | 3.2 ± 4.9 |
| Moderate | silodosin | 128 | 10.3 ± 3.0 | 2.3 ± 5.3. |

As shown in Table 5, the changes after 52 weeks of silodosin administration were 3.2 mL/sec in the severe group and 2.3 mL/sec in the moderate group, and the improvement of the objective symptom was observed in each group in comparison with the case of before administration. Particularly, the effect was significant in the severe group.

### Industrial Applicability

The pharmaceutical composition is markedly useful as an agent for the prevention of transition to surgical therapy, because it can significantly improve subjective and objective symptoms of patients who are subject to surgical therapy for benign prostatic hyperplasia.

## Claims

1. A pharmaceutical composition for oral administration, for use in the prevention of transition to surgical therapy for benign prostatic hyperplasia in a patient of benign prostatic hyperplasia whose overall severity is severe, which comprises as daily dose per adult 4 to 8 mg of silodosin or a pharmaceutically acceptable salt thereof.

2. A pharmaceutical composition for use as claimed in claim 1 wherein the surgical therapy is transurethral resection of the prostate.

3. A pharmaceutical composition for use as claimed in claim 1 or 2 wherein the prevention comprises administering the silodosin or the pharmaceutically acceptable salt thereof to a patient of benign prostatic hyperplasia whose overall severity is severe.

4. A pharmaceutical composition for oral administration for use in treating a patient of benign prostatic hyperplasia whose overall severity is severe, which comprises as daily dose per adult 4 to 8 mg of silodosin or a pharmaceutically acceptable salt thereof.

5. Use of silodosin or a pharmaceutically acceptable salt thereof in the manufacture of a pharmaceutical composition for oral administration comprising as daily dose 4 to 8 mg per adult of the silodosin or the pharmaceutically acceptable salt thereof for preventing the transition to surgical therapy for benign prostatic hyperplasia in a patient of benign prostatic hyperplasia whose overall severity is severe or for treating a patient of benign prostatic hyperplasia whose overall severity is severe.

6. Use as claimed in claim 5 wherein the surgical therapy is transurethral resection of the prostate.

## Patentansprüche

1. Pharmazeutische Zusammensetzung zur oralen Verabreichung zur Verwendung bei der Vorbeugung des Übergangs zur chirurgischen Therapie der benignen Prostata-Hyperplasie bei einem Patienten mit bemgner Prostata-Hyperplasie, deren Gesamt-Schweregrad hoch ist, die als Tagesdosis für Erwachsene 4 bis 8 mg Silodosin oder eines pharmazeutisch akzeptablen Salzes davon enthält

2. Pharmazeutische Zusammensetzung zur Verwendung wie in Anspruch 1 beansprucht, wobei es sich bei der chirurgischen Therapie um die transurethrale Resektion der Prostata handelt.

3. Pharmazeutische Zusammensetzung zur Verwendung wie in Anspruch 1 oder 2 beansprucht, wobei die Vorbeugung die Verabreichung des Silodosins oder des pharmazeutisch akzeptablen Salzes davon an einen Patienten mit benigner Prostata-Hyperplasie, deren Gesamt-Schweregrad hoch ist, umfasst.

4. Pharmazeutische Zusammensetzung zur oralen Verabreichung zur Verwendung bei der Behandlung eines Patienten mit benigner Prostata-Hyperplasie, deren Gesamt-Schweregrad hoch ist, die als Tagesdosis für Erwachsene 4 bis 8 mg Silodosin oder eines pharmazeutisch akzeptablen Salzes davon enthält.

5. Verwendung von Silodosin oder eines pharmazeutisch akzeptablen Salzes davon bei der Herstellung einer pharmazeutischen Zusammensetzung zur oralen Verabreichung, die als Tagesdosis für Erwachsene 4 bis 8 mg Silodosin oder des pharmazeutisch akzeptablen Salzes davon enthält, zur Vorbeugung des Übergangs zur chirurgischen Therapie der benignen Prostata-Hyperplasie bei einem Patienten mit benigner Prostata-Hyperplasie, deren Gesamt-Schweregrad hoch ist, oder zur Behandlung eines Patienten mit benigner Prostata-Hyperplasie, deren Gesamt-Schweregrad hoch ist.

6. Verwendung wie in Anspruch 5 beansprucht, wobei es sich bei der chirurgischen Therapie um die transurethrale Resektion der Prostata handelt.

## Revendications

1. Composition pharmaceutique pour l'administration orale, pour utilisation dans la prévention de la transition à une thérapie chirurgicale pour une hyperplasie prostatique bénigne chez un patient atteint d'hyperplasie prostatique bénigne, dont la sévérité dans l'ensemble est sévère, qui comprend comme dose journalière par adulte 4 à 8 mg de silodosine ou un sel pharmaceutiquement acceptable de celle-ci.

2. Composition pharmaceutique pour utilisation selon la revendication 1, dans laquelle la thérapie chirurgicale est la résection trans-urétrale de la prostate

3. Composition pharmaceutique pour utilisation selon la revendication 1 ou 2, dans laquelle la prévention comprend l'administration de la silodosine ou d'un sel pharmaceutiquement acceptable de celle-ci à un patient atteint d'hyperplasie prostatique bénigne dont la sévérité dans 1 ensemble est sévère

4. Composition pharmaceutique pour administration, orale pour utilisation dans le traitement d'un patient atteint d'hyperplasie prostatique bénigne dont la severite dans l'ensemble est sévère, qui comprend comme dose journalière par adulte 4 à 8 mg de silodosine ou un sel pharmaceutiquement acceptable de celle-ci

5. Utilisation de silodosine ou d'un sel pharmaceutiquement acceptable de celle-ci dans la fabrication d'une composition pharmaceutique pour l'administration orale comprenant comme dose journalière 4 à 8 mg par adulte de la silodosine ou d'un sel pharmaceutiquement acceptable de celle-ci pour la prévention de la transition à la thérapie chirurgicale pour l'hyperplasie prostatique bénigne chez un patient atteint d'hyperplasie prostatique bénigne dont la sévérité dans l'ensemble est sévère ou pour le traitement d'un patient atteint d'hyperplasie prostatique bénigne dont la sévérité dans l'ensemble est sévère.

6. Utilisation selon la revendication 5, dans laquelle la thérapie chirurgicale est la résection trans-urétrale de la prostate.
